# EUROPÄISCHE PATENTSCHRIFT

(11) **EP 2 194 035 B1**
(45) Veröffentlichungstag und Bekanntmachung des Hinweises auf die Patenterteilung: **02.11.2011**
(21) Anmeldenummer: 09177828.2
(22) Anmeldetag: 03.12.2009
(51) Int. Cl.: C07C 45/00

(54) **Hydroxytetralone**
Hydroxytetralones
Hydroxytétralone

(30) Priorität: 06.12.2008 DE 102008060958
(43) Veröffentlichungstag der Anmeldung: 09.06.2010
(73) Patentinhaber: Saltigo GmbH, 40764 Langenfeld (DE)
(72) Erfinder: Kreis, Michael, 51373, Leverkusen (DE); Schlummer, Björn, 53115, Bonn (DE); Mindach, Olaf, 51379, Leverkusen (DE)
(74) Vertreter: Wichmann, Birgid

(56) Entgegenhaltungen:
- EP-A1- 0 889 019
- DE-A1- 2 219 116
- JP-A- 2004 277 326
- OSTASHEVSKAYA L ET AL: "Ionic Hydrogenation of Dihydroxynaphthalenes with Cyclohexane in the Presence of Aluminum Bromide" RUSSIAN JOURNAL OF ORGANIC CHEMISTRY, CONSULTANTS BUREAU, US, Bd. 36, Nr. 10, 1. Januar 2000 (2000-01-01), Seiten 1474-1477, XP008117372 ISSN: 1070-4280

## Beschreibung

Die vorliegende Erfindung betrifft ein Verfahren zur Herstellung von gegebenenfalls substituierten Hydroxytetralonen.

Hydroxytetralone besitzen Bedeutung als Intermediate für die Synthese von Arzneimitteln, Agrochemikalien und Feinchemikalien. Insbesondere werden Hydroxytetralone als Intermediate zur Herstellung von Antiphlogistika und Antidepressiva verwendet.

Verschiedene Verfahren zur Herstellung von Hydroxytetralonen sind bekannt.

In Russ. J. Org. Chem. 2000, 36, 1474-1477 wird die Herstellung von Hydroxytetralonen durch Reaktion von Dihydroxynapthalin mit Aluminiumbromid in Dibrommethan beschrieben. Nachteilig bei diesem Verfahren ist die Verwendung von Aluminiumbromid, da dieses schlecht handhabbar und ökotoxikologisch problematisch ist.

Aus Tetrahedron Lett., 1983, 24, 3095-3098, ist die Herstellung von Hydroxytetralonen durch Oxidation von 2-Tetralon mit Wasserstoffperoxid in supersauren Medien bekannt. Das Verfahren bietet lediglich eine geringe Produktselektivität und ist aus toxikologischen und ökonomischen Gründen bedenklich.

In Tetrahedron 2000, 56, 5353-5361 wird ein Festphasenverfahren zur Herstellung von Hydroxytetralonen beschrieben. Aus ökonomischen Gründen ist auch dieses Verfahren ungeeignet.

Alternativ dazu erfolgt in der JP 2005289981 A die Synthese ausgehend von Dihydroxynaphtalin durch Transferhydrierung mit Kaliumformiat, was Nachteile in der Wirtschaftlichkeit aufweist.

Die Herstellung von einem Hydroxytetralon wird in der JP 2004277326 A durch Hydrierung von Dihydroxynaphtalin in polaren Lösungsmitteln mit Basen unter Verwendung von Palladium/Silber-Katalysatoren beschrieben. Auch dieses Verfahren ist unökonomisch, da größere Mengen teurer Palladium/Silber Katalysatoren eingesetzt werden müssen.

Die bekannten Verfahren weisen die Nachteile auf, dass sie entweder unökonomisch, ökologisch bedenklich oder sicherheitstechnisch problematisch sind.

Es bestand daher die Aufgabe, ein Verfahren bereitzustellen, dass die Nachteile der bisherigen Verfahren nicht aufweist und die Hydroxytetralone in guten Ausbeuten, kostengünstig in technischem Maßstab liefert.

Überraschenderweise wurde gefunden, dass mit dem erfindungsgemäßen Verfahren, unter Verwendung von speziellen, organischen, unpolaren, zyklischen Lösungsmitteln die Nachteile des Standes der Technik beseitigt werden konnten und Hydroxytetralone in guten Ausbeuten hergestellt werden konnten.

Gegenstand der Erfindung ist daher ein Verfahren zur Herstellung von Verbindungen der Formel (I) 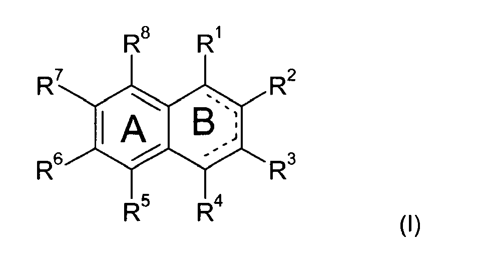 wobei Ring B vollständig oder teilweise hydriert ist und R¹, R², R³, R⁴, R⁵, R⁶, R⁷ oder R⁸ gleich und verschieden sein können und mindestens einer der Reste am Ring A und am Ring B für Hydroxy steht und die anderen Reste ausgewählt sind aus der Gruppe C₁-C₁₅-Alkyl, besonders bevorzugt C₁-C₆-Alkyl, C₆-C₁₂-Aryl, C₇-C₁₅-Arylalkyl, C₁-C₁₅-Alkylthio, C₁-C₁₅-Alkoxy, besonders bevorzugt C₁-C₆-Alkoxy, Hydroxy, Wasserstoff, C₁-C₁₀-Mono- bzw. C₁-C₁₀-Dialkylamino, C₁-C₁₅-Halogenalkyl, C₁-C₁₅-Halogenalkoxy, C₁-C₁₅Halogenalkylthio, mit Halogen = F, Cl, Br, I, die gegebenenfalls weiter substituiert sein können,
**dadurch gekennzeichnet, dass** Verbindungen der Formel (II) 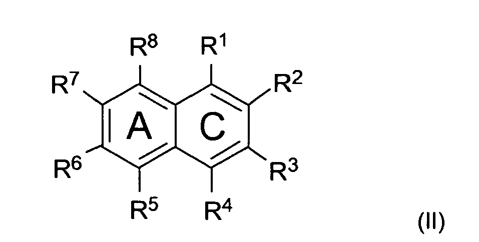 wobei R¹, R², R³, R⁴, R⁵, R⁶, R⁷ und R⁸ die vorstehende Bedeutung aufweisen und mindestens einer der Reste am Ring A und am Ring C für Hydroxy stehen,
in Gegenwart von mindestens einem Übergangsmetall-Katalysator,
mindestens einem organischen, unpolaren, alizyklischen oder aromatischen Lösungsmittel und Wasserstoff umgesetzt werden.

Es ist durchaus möglich, dass die am Ring B gebundenen Hydroxylgruppen der teilhydrierten Verbindungen der Formel (I) in der Form eines Enols vorliegen und der Keto-Enol Tautomerie unterliegen. Daher sind von der Erfindung und ihrer Bedeutung nach, die durch die Keto-Enol Tautomerie am Ring B der teilhydrierten Verbindungen der Formel (I) gebildeten Ketone, wie z.B. 7-Hydroxy-2-tetralon, ebenfalls umfasst.

In der vorliegenden Anmeldung entspricht die Nummerierung der Ringkohlenstoffatome der Verbindungen der Formel (I) und (II) den Indizes des jeweiligen Restes R. Also ist z.B. der Rest R¹ am C₁-Kohlenstoff des Ringes B gebunden und z.B. der Rest R⁵ am C₅-Kohlenstoff des Ringes A gebunden.

Alkyl bzw. Alkoxy bzw. Alkylthio steht im Rahmen der Erfindung für einen geradkettigen, zyklischen, verzweigten oder unverzweigten Alkyl- bzw. Alkoxy- bzw. Alkylthio- Rest mit 1 bis 15 (C₁-C₁₅), bevorzugt 1 bis 12 (C₁-C₁₂ und besonders bevorzugt 1 bis 6 (C₁C₆). Beispielhaft steht Alkyl für Methyl, Ethyl, n-Propyl, Isopropyl, n-, i-, s- oder t-Butyl, Cyclopropyl, Cyclobutyl, Cyclopentyl, Cyclohexyl, Cycloheptyl, n-Pentyl, 1-Methylbutyl, 2-Methylbutyl, 3-Methylbutyl, neo-Pentyl, 1-Ethylpropyl, cyclo-Hexyl, cyclo-Pentyl, n-Hexyl, n-Heptyl, n-Octyl, n-Decyl und n-Dodecyl. Vorzugsweise steht Alkyl für Methyl, Ethyl, n-Propyl, Isopropyl, n-, i-, s- oder t-Butyl, Cyclopropyl, Cyclobutyl, Cyclopentyl, n-Hexyl, Cyclohexyl, n-Pentyl, 1-Methylbutyl, 2-Methylbutyl, 3-Methylbutyl, neo-Pentyl, 1-Ethylpropyl, cyclo-Hexyl. Besonders bevorzugt steht Alkyl für Methyl, Ethyl, n-Propyl, Isopropyl, n-, i-, s- oder t-Butyl, n-Pentyl und n-Hexyl. Beispielhaft und vorzugsweise steht Alkoxy für Methoxy, Ethoxy, n-Propoxy, Isopropoxy, t-Butoxy, n-Pentoxy und n-Hexoxy. Beispielhaft und vorzugsweise steht Alkylthio für Methanthiol, Ethanthiol, n-Propanthiol, Isopropanthiol, n-, i-, s- oder t-Butanthio, n-Pentanthio, 1-Methylbutanthio, 2-Methylbutanthio, 3-Methylbutanthio, neo-Pentanthio, 1-Ethylpropanthio und n-Hexanthio.

Aryl steht im Rahmen der Erfindung für einen monocarbozyklischen aromatischen Rest mit vorzugsweise 6 bis 12 aromatischen Kohlenstoffatomen (C₆-C₁₂-Aryl). Die carbozyklischen aromatischen Reste können mit bis zu fünf gleichen oder verschiedenen Substituenten pro Zyklus substituiert sein, wie z.B. Alkyl, Alkoxy, Aryl, Arylalkyl, Dialkylamino, Halogen, Halogenalkyl, Halogenalkoxy, Halogenalkylthio und Monoalkylamino. Beispielhaft und bevorzugt steht C₆-C₁₂-Aryl für Phenyl und Biphenyl.

Mono- bzw. Dialkylamino stehen im Rahmen der Erfindung für eine Amino-Gruppe mit einem oder zwei gleichen oder verschiedenen, zyklischen, geradkettigen oder verzweigten Alkylsubstituenten, die vorzugsweise jeweils 1 bis 10, besonders bevorzugt 1 bis 7 (C₁-C₇) Kohlenstoffatome aufweisen. Beispielhaft und vorzugsweise steht Monoalkylamino für Methylamino, Ethylamino, n-Propylamino, Isopropylamino, t-Butylamino, n-Pentylamino und n-Hexylamino sowie Dialkylamino für *N,N*-Dimethylamino, *N*,*N*-Diethylamino, *N-*Ethyl-*N-*methylamino, *N*-Methyl-*N*-n-propylamino, *N*-Isopropyl-*N*-n-propylamino, *N*-t-Butyl-*N*-methylamino, *N*-Ethyl-*N*-n-pentylamino und *N*-n-Hexyl-*N*-methylamino.

Arylalkyl bedeutet jeweils unabhängig voneinander einen geradkettigen, zyklischen, verzweigten oder unverzweigten Alkyl-Rest nach vorstehender Definition, der einfach, mehrfach oder vollständig durch Aryl-Reste gemäß vorstehender Definition substituiert sein kann. Ein Beispiel für Arylalkyl ist Benzyl. Bevorzugt sind Arylalkyle mit 7 bis 15 (C₇-C₁₅) Kohlenstoffatome, besonders bevorzugt sind Arylalkyle mit 7 bis 10 (C₇-C₁₀) Kohlenstoffatomen.

Halogenalkyl bzw. Halogenalkoxy steht im Rahmen der Erfindung für einen geradkettigen, zyklischen, verzweigten oder unverzweigten Alkyl-, bzw. Alkoxy-Rest nach vorstehender Definition, der einfach, mehrfach oder vollständig mit Halogenatomen substituiert ist. Beispielhaft und vorzugsweise steht Halogenalkyl für Chlorethyl, Chlorpropyl, Dichlormethyl, Difluormethyl, Fluormethyl, Fluorethyl, Fluorpropyl, Trifluormethyl und 2,2,2-Trifluorethyl und Halogenalkoxy für Difluormethoxy, Fluorethoxy, Fluormethoxy, Trifluormethoxy, Trichlormethoxy und 2,2,2-Triffluorethoxy.

Halogenalkylthio bedeutet im Rahmen der Erfindung einen geradkettigen, zyklischen, verzweigten oder unverzweigten Rest mit 1 bis 15 Kohlenstoffatomen, bevorzugt mit 1 bis 6 (C₁-C₆) Kohlenstoffatomen, der einfach, mehrfach oder vollständig mit Halogenatomen substituiert ist. Beispielhaft und vorzugsweise steht Halogenalkylthio für Chlorethylthio, Chlorbutylthio, Chlorhexylthio, Chlorpentylthio, Chlordodecylthio, Dichlorethylthio, Fluorethylthio, Trifluormethylthio und 2,2,2-Trifluorethylthio.

Besonders bevorzugt sind die Reste R¹, R², R³, R⁴, R⁵, R⁶, R⁷ oder R⁸ gleich und verschieden und stehen für (C₁-C₆)-Alkyl, (C₆-C₁₂)-Aryl, (C₇-C₁₀)-Arylalkyl, (C₁-C₆)-Alkoxy, Hydroxy, (C₁-C₆)-Halogenalky, (C₁-C₆)-Halogenalkoxy, (C₁-C₆)-Alkylthio, (C₁-C₆)-Halogenalkylthio und Wasserstoff.

Beispielsweise und vorzugsweise ist Ring A der Verbindungen der Formel (II) durch eine, zwei oder drei Hydroxylgruppen substituiert und Ring B der Verbindungen der Formel (II) durch eine, zwei oder drei Hydroxylgruppen substituiert, bevorzugt ist Ring A der Verbindungen der Formel (II) durch eine oder zwei Hydroxylgruppen in 6 oder/und 7 Position substituiert und Ring B der Verbindungen der Formel (II) durch eine oder zwei Hydroxylgruppen in 2 oder/und 3 Position.

Beispielsweise und vorzugsweise ist in Ring B der Verbindungen der Formel (I) zwischen den Kohlenstoffatomen 1 und 2, 2 und 3, oder 3 und 4 nach der Hydrierung noch eine oder keine Doppelbindung mehr vorhanden. Besonders bevorzugt ist Ring B der Verbindungen der Formel (I) teilhydriert, d.h. nach der Hydrierung ist noch eine Doppelbindung vorhanden.

Besonders bevorzugt stehen die Verbindungen der Formel (I) für 1,2,3,4-Tetrahydro-7-hydroxynaphthyl-1-on, 1,2,3,4-Tetrahydro-7-hydroxynaphthyl-2-o n , 1 , 2 , 3 , 4-Tetrahydro-6-hydroxynaphthyl-1-on, 1,2,3,4-Tetrahydro-6-hydroxynaphthyl-2-on 1,2,3,4-Tetrahydro-5-hydroxynaphthyl-1-o n , 1 , 2 , 3 , 4-Tetrahydro-5-hydroxynaphthyl-2-o n , 1 , 2 , 3 , 4-Tetrahydro-8-hydroxynaphthyl-1-on und 1,2,3,4-Tetrahydro-8-hydroxynaphthyl-2-on.

Der Rahmen der Erfindung umfasst alle oben stehenden und im Folgenden aufgeführten, allgemeinen oder in Vorzugsbereichen genannten Restedefinitionen, Parameter und Erläuterungen untereinander, also auch zwischen den jeweiligen Bereichen und Vorzugsbereichen in beliebiger Kombination.

Als Lösungsmittel zur Durchführung des erfindungsgemäßen Verfahrens eignen sich insbesondere organische, unpolare alizyklische oder aromatische Lösungsmittel, gegebenenfalls halogenierte Kohlenwasserstoffe, wie beispielsweise Benzol, Toluol, Xylol, Chlorbenzol, Dichlorbenzol, Cyclohexan, Cyclopentan, Cycloheptan, Cyclononan, Cyclooctan, Methylcyclopentan, Methylcyclohexan, Bicyclo-[4.1.0]-heptan oder Gemische solcher Lösungsmittel. Besonders bevorzugte Lösungsmittel sind Toluol, Xylol, Benzol, Cyclohexan und Methylcyclohexan oder Gemische dieser Lösungsmittel.

In einer weiteren bevorzugten Ausführungsform der Erfindung ist das Übergangsmetall des Übergangsmetall-Katalysators Rhodium, Ruthenium, Iridium, Platin, Palladium oder deren Salze, besonders bevorzugt sind Rhodium, Ruthenium, Platin, Palladium oder deren Salze und Gemische solcher Übergangsmetalle oder deren Salze.

In einer anderen Ausführungsform des erfindungsgemäßen Verfahrens befindet sich das Übergangsmetall bevorzugt auf einem Träger, ausgewählt aus der Gruppe Siliziumdioxid, Aluminiumoxid, Titandioxid, Zeolithe oder Kohlenstoff. Ganz besonders bevorzugt handelt es sich bei dem Träger um Aktivkohle oder Titandioxid.

In den Fällen, in denen die Übergangsmetall-Katalysatoren anorganisch geträgerte Katalysatoren darstellen, sind Ru-Titandioxid, Ru-Siliziumdioxid, Ru-Aluminiumdioxid, Ru-Kohlenstoff, Pd-Kohlenstoff, Pd-Aktivkohle, P d-Titandioxid, Pd-Siliziumdioxid, Pd-Aluminiumdioxid, Rh-Titandioxid, Rh-Siliziumdioxid, Rh-Kohlenstoff, Ir-Kohlenstoff, Ir-Titandioxid, Pt-Kohlenstoff, Pt-Aktivkohle, Pt-Titandioxid, Pt-Aluminiumoxid und/ oder Pt-Siliziumdioxid bevorzugt. Besonders bevorzugt werden als Übergangsmetall-Katalysatoren Pd-Kohlenstoff, P d-Titandioxid, Pd-Siliziumdioxid, Pd-Aluminiumdioxid, Pt-Kohlenstoff, Rh-Kohlenstoff und Ru-Kohlenstoff, ganz besonders bevorzugt Pd-Aktivkohle, Pd-Titandioxid, Pt-Titandioxid oder Pt-Aktivkohle eingesetzt.

Die Übergangsmetall-Katalysatoren können beispielsweise als makroskopischer Festkörper oder als Partikel z.B. in Form einer kolloidalen Lösung eingesetzt. Beispielsweise und vorzugsweise weisen die Übergangsmetall-Katalysatoren einen Wassergehalt von 1 - 90 %, besonders bevorzugt von 20 - 80 % und ganz besonders bevorzugt von 40 - 60 % auf.

Bei den eingesetzten geträgerten oder nicht geträgerten Übergangsmetallkatalysatoren handelt es sich um handelsübliche Produkte.

In einer Ausführungsform der Erfindung liegt die Menge des Übergangsmetalls in den Übergangsmetall-Katalysatoren bezogen auf die eingesetzte Masse der geträgerten Übergangsmetall-Katalysatoren, zwischen 0,01 und 99 Gew. %. Bevorzugt liegt die Konzentration zwischen 1 und 60 Gew. %. Besonders bevorzugt zwischen 1 und 10 Gew. %.

Die Menge der eingesetzten Übergangsmetall-Katalysatoren kann so gewählt werden, dass die Menge an Übergangsmetall, bezogen auf die eingesetzte Menge der Verbindungen der Formel (II), zwischen 0.01 und 10 mol % bevorzugt zwischen 0.02 und 8 mol % und besonders bevorzugt zwischen 0.5 und 5 mol % liegt.

In einer weiteren besonders bevorzugten Ausführungsform der Erfindung liegt die Menge der eingesetzten Übergangsmetall-Katalysatoren bezogen auf die eingesetzte Menge der Verbindungen der Formel (II) zwischen 0.5 Gew. % und 5 Gew. %.

In dem erfindungsgemäßen Verfahren kann der Wasserstoff beispielsweise in elementarer Form der Reaktionsmischung zugeführt werden. Der Wasserstoff kann aber auch in situ aus anderen Wasserstoffquellen, wie beispielsweise Hydrazin, Wasserstoffperoxid, Lithiumalanat oder Natriumborhydrid hergestellt werden. Bevorzugt wird der Wasserstoff außerhalb der Reaktionsmischung erzeugt und zugeführt. Beispielsweise und vorzugsweise können 1 bis 10 Moläquivalente Wasserstoff bezogen auf die Menge der Verbindungen der Formel (II) zugegeben werden, bevorzugt werden 1 bis 5 Moläquivalente zugegeben, besonders bevorzugt werden 1 bis 2 Moläquivalente bezogen auf die Menge der Verbindungen der Formel (II) zugegeben.

In einer weiteren Ausführungsform der Erfindung wird die Reaktion in Gegenwart von Puffersubstanzen durchgeführt. Beispielsweise und vorzugsweise sind diese Puffersubstanzen ausgewählt aus der Gruppe Acetat, Phosphat, Borat, Carbonat, Hydrogencarbonat oder Hydrogenphosphat. Bevorzugt ist die Puffersubstanz Alkali- oder Erdalkalimetaltetraborat, besonders bevorzugt ist die Puffersubstanz Natriumtetraborat (Borax).

Beispielsweise und vorzugsweise kann die Menge der Puffersubstanz bezogen auf die Verbindungen der Formel (II) 0.001 Mol % bis 10 Mol % betragen, bevorzugt 0.01 Mol % bis 1 Mol % und ganz besonders bevorzugt 0.01 bis 0.5 Mol %.

Die Reaktionstemperatur liegt vorzugsweise zwischen 80°C und 200°C, besonders bevorzugt zwischen 100 °C und 180°C, ganz besonders bevorzugt zwischen 120°C und 170°C.

Die Reaktion wird vorzugsweise bei Drücken zwischen 1 und 200 bar, besonders bevorzugt zwischen 10 und 100 bar, ganz besonders bevorzugt zwischen 20 und 80 bar durchgeführt.

Viele der Verbindungen der Formel (II) sind handelsüblich. Die Herstellung der Verbindungen der Formel (II) kann nach analogen, aus dem Stand der Technik bekannten Verfahren erfolgen und ist dem Durchschnittsfachmann bekannt.

In einer Ausführungsform der Erfindung wird zunächst die Verbindung der Formel (II) mit den organischen, unpolaren, alizyklischen oder aromatischen Lösungsmitteln in Kontakt gebracht und dann der Übergangsmetall-Katalysator zugegeben. Ebenfalls möglich ist auch die Übergangsmetall-Katalysatoren gleich zur Reaktionsmischung zuzugeben. Beispielsweise ist es ebenfalls möglich zunächst die Verbindungen der Formel (II) mit den organischen, unpolaren, alizyklischen oder aromatischen Lösungsmitteln in Kontakt zu bringen und dann die Puffersubstanz hinzuzufügen. Ebenfalls ist es aber auch möglich beispielsweise zunächst die Puffersubstanz in dem organischen, unpolaren, alizyklischen oder aromatischen Lösungsmitteln vorzulegen und dann die Verbindungen der Formel (II) hinzuzufügen.

Bevorzugt wird die Reaktion in einem Autoklaven durchgeführt. Des Weiteren ist bevorzugt unter inerten Bedingungen zu arbeiten. Die Inertisierung kann z.B. durch kontinuierliche oder diskontinuierliche Durchströmung der Reaktionsmischung mit inerten Gasen, wie z.B. Argon und Stickstoff, durchgeführt werden. Anschließend wird Wasserstoff aufgedrückt und z.B. bis zur Druckkonstanz hydriert. Ebenso kann die Reaktion aber auch schon früher beendet werden und dadurch teilhydrierte Produkte hergestellt werden. Bevorzugt wird die Reaktion beendet, wenn 1 bis 2 Moläquivalente Wasserstoff bezogen auf die Konzentration der Verbindung der Formel (II) reagiert haben, besonders bevorzugt wenn 1,1 bis 1,3 Moläquivalente Wasserstoff reagiert haben.

In einer besonders bevorzugten Ausführungsform wird die Verbindung der Formel (II) mit den Lösungsmitteln in Kontakt gebracht. Danach werden die Übergangsmetall-Katalysatoren zugegeben. Im Anschluss daran wird Natriumtetraborat zugegeben, der Autoklav bzw. die Reaktionsmischung inertisiert, das Reaktionsgemisch erwärmt und erst in der letzten Stufe Wasserstoff aufgedrückt und solange hydriert bis zwischen 1,1 und 1,3 Moläquivalente Wasserstoff reagiert haben.

Auf erfindungsgemäße Weise können die Verbindungen der Formel (I) unter Verwendung kostengünstiger Materialien in kurzer Zeit, d.h. sehr wirtschaftlich in guten Ausbeuten in technischen Prozessen hergestellt werden. Die Aufarbeitung kann in an sich bekannter Weise, z.B. durch Extraktion mit bekannten Lösungsmitteln, wie beispielsweise Aceton oder Diethylether und nachfolgender Filtration und Destillation erfolgen.

Die erfindungsgemäß hergestellten Verbindungen der Formel (I) eignen sich insbesondere als Zwischenprodukte z.B. zur Herstellung von Arzneimitteln, Agrochemikalien und Feinchemikalien.

Die nachstehenden Beispiele dienen der Erläuterung der Erfindung, ohne dabei auf diese beschränkt zu sein.

### Beispiele

### 1. Herstellung von 7-Hydroxy-2-tetralon in Methylcyclohexan

3,00 g (18,5 mmol) 2,7-Dihydroxynaphtalin, 20 mg Palladium auf Kohle (5 Gew. % Palladium auf Kohle) und 9 mg Natriumtetraborat (Borax) (0,04 mmol) werden in 6 g Methylcyclohexan suspendiert. Der Reaktor wird inertisiert und 2 bar Wasserstoff aufgedrückt. Dann wird das Reaktionsgemisch auf 200°C aufgeheizt und 30 Minuten thermostatisiert. Der Reaktor wird auf 155°C thermostatisiert und 80 bar aufgedrückt. Die Reaktion wird so lange gerührt, bis ca. 1,2 Äquivalente Wasserstoff aufgedrückt sind. Der Reaktor wird auf Raumtemperatur abgekühlt und auf Normaldruck entspannt. Die Reaktionssuspension wird dreimal mit jeweils 10 ml Aceton gerührt und über Celite abfiltriert. Lösungsmittel werden unter vermindertem Druck destillativ entfernt. Der erhaltene Feststoff wird über Nacht im Vakuumtrockenschrank bei 50°C und 200 mbar getrocknet, dabei werden 3,01 g hellgelbe Kristalle mit einer Zusammensetzung von 65,1% 7-Hydroxy-2-tetralon, 20,6% 7-Hydroxy-2-tetralol und 10,1% 2,7-Dihydroxynaphthalin erhalten.

### 2. Herstellung von 7-Hydroxy-2-tetralon in Toluol

3,0 g (18,5 mmol) 2,7-Dihydroxynaphthalin, 90 mg Palladium auf Kohle (5 Gew. % Palladium auf Kohle) und 9 mg Natriumtetraborat (Borax) (0,04 mmol) werden in 6 g Toluol suspendiert. Der Reaktor wird inertisiert und 2 bar Wasserstoff aufgedrückt. Der Reaktor wird auf 120°C thermostatisiert und 20 bar aufgedrückt. Die Reaktion wird so lange gerührt, bis ca. 1,2 Äquivalente Wasserstoff aufgedrückt sind. Der Reaktor wird auf Raumtemperatur abgekühlt und auf Normaldruck entspannt. Die Reaktionssuspension wird dreimal mit jeweils 5 ml Aceton gerührt und über Celite abfiltriert. Lösungsmittel werden unter vermindertem Druck destillativ entfernt. Der erhaltene Feststoff wird über Nacht im Vakuumtrockenschrank bei 50°C und 200 mbar getrocknet, dabei werden 2,95 g hellgelbe Kristalle mit einer Zusammensetzung von 67% 7-Hydroxy-2-tetralon, 20% 7-Hydroxy-2-tetralol und 11% 2,7-Dihydroxynaphtalin erhalten.

### 3. Herstellung von 7-Hydroxy-2-tetralon in Xylol

3,0 g (18,5 mmol) 2,7-Dihydroxynaphthalin, 90 mg Palladium auf Kohle (5 Gew. % Palladium auf Kohle) und 9 mg Natriumtetraborat (Borax) (0,04 mmol) werden in 6 g Toluol suspendiert. Der Reaktor wird inertisiert und 2 bar Wasserstoff aufgedrückt. Der Reaktor wird auf 120°C thermostatisiert und 40 bar aufgedrückt. Die Reaktion wird so lange gerührt, bis ca. 1,2 Äquivalente Wasserstoff aufgedrückt sind. Der Reaktor wird auf Raumtemperatur abgekühlt und auf Normaldruck entspannt. Die Reaktionssuspension wird dreimal mit jeweils 5 ml Aceton gerührt und über Celite abfiltriert. Lösungsmittel werden unter vermindertem Druck destillativ entfernt. Der erhaltene Feststoff wird über Nacht im Vakuumtrockenschrank bei 50°C und 200 mbar getrocknet, dabei werden 2,97 g hellgelbe Kristalle mit einer Zusammensetzung von 67% 7-Hydroxy-2-tetralon, 21 % 7-Hydroxy-2-tetralol und 11% 2,7-Dihydroxynaphthalin erhalten.

### Vergleichsbeispiel

### 1. Herstellung von 7-Hydroxy-2-tetralon in Ethanol

3,0 g (18,5 mmol) 2,7-Dihydroxynaphthalin, 60 mg Palladium auf Kohle (5 Gew. % Palladium auf Kohle) und 6 mg Natriumtetraborat (Borax) (0,03 mmol) werden in 6 g Ethanol suspendiert. Der Reaktor wird inertisiert und 2 bar Wasserstoff aufgedrückt. Der Reaktor wird auf 160°C thermostatisiert und 10 bar aufgedrückt. Die Reaktion wird 16 h gerührt. Dabei findet jedoch keine Wasserstoffaufnahme statt. Der Reaktor wird auf Raumtemperatur abgekühlt und auf Normaldruck entspannt. Die Reaktionssuspension wird dreimal mit jeweils 5 ml Aceton gerührt und über Celite abfiltriert. Analytik der Rohlösung zeigt keine Hydrierprodukte sondern lediglich 2,7-Dihydroxynaphthalin.

## Patentansprüche

1. Verfahren zur Herstellung von Verbindungen der Formel (I) 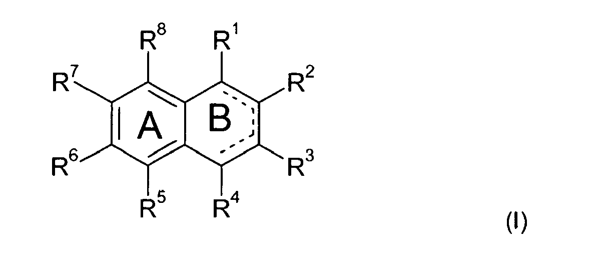 wobei Ring B vollständig oder teilweise hydriert ist und
R¹, R², R³, R⁴, R⁵, R⁶, R⁷ oder R⁸ gleich und verschieden sein können und mindestens einer der Reste am Ring A und am Ring B für Hydroxy steht und die anderen Reste ausgewählt sind aus der Gruppe C₁-C₁₅-Alkyl, C₆-C₁₂-Aryl, C₇-C₁₅-Arylalkyl, C₁-C₁₅-Alkylthio, C₁-C₁₅-Alkoxy, Hydroxy, Wasserstoff, C₁-C₁₀-Mono- bzw. C₁-C₁₀-Dialkylamino, C₁-C₁₅-Halogenalkyl, C₁-C₁₅-Halogenalkoxy, C₁-C₁₅-Halogenalkylthio, mit Halogen = F, Cl, Br, I, die gegebenenfalls weiter substituiert sein können,
**dadurch gekennzeichnet, dass** Verbindungen der Formel (II) 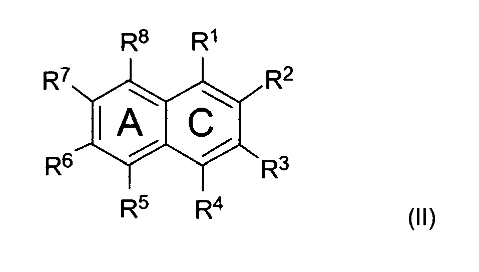 wobei R¹, R², R³, R⁴, R⁵, R⁶, R⁷ und R⁸ die vorstehende Bedeutung aufweisen und mindestens einer der Reste am Ring A und am Ring C für Hydroxy steht,
in Gegenwart von mindestens einem Übergangsmetall-Katalysator,
mindestens einem organischen, unpolaren, alizyklischen oder aromatischen Lösungsmittel und Wasserstoff umgesetzt werden.

2. Verfahren nach Anspruch 1, **dadurch gekennzeichnet, dass** R₁, R₂, R₃, R₄, R₅, R₆, R₇ oder R₈ gleich und verschieden sein können und für (C₁-C₆)-Alkyl, (C₆-C₁₂)-Aryl, (C₇-C₁₅)-Arylalkyl, (C₁-C₆)-Alkoxy, Hydroxy, (C₁-C₆)-Halogenalkyl, (C₁-C₆)-Halogenalkoxy, (C₁-C₆)-Halogenalkylthio, (C₁-C₆)-Alkylthio oder Wasserstoff stehen.

3. Verfahren nach einem der Ansprüche 1 bis 2, **dadurch gekennzeichnet, dass** Ring A der Verbindungen der Formel (I) durch eine oder zwei Hydroxylgruppen in 6 und/oder 7 Position substituiert ist und Ring B der Verbindungen der Formel (I) durch eine oder zwei Hydroxylgruppen in 2 und/oder 3 Position substituiert ist.

4. Verfahren nach einem der Ansprüche 1 bis 3, **dadurch gekennzeichnet, dass** Ring B der Verbindungen der Formel (I) teilhydriert ist und zwischen den Kohlenstoffatomen 1 und 2, 2 und 3, oder 3 und 4 nach der Hydrierung noch eine Doppelbindung vorhanden ist.

5. Verfahren nach einem oder mehreren Ansprüchen 1 bis 4, **dadurch gekennzeichnet, dass** es sich bei den organischen, unpolaren alizyklischen oder aromatischen Lösungsmitteln um Benzol, Toluol, Xylol, Cyclohexan, oder Gemische solcher organischen Lösungsmittel handelt.

6. Verfahren nach einem oder mehreren der Ansprüche 1 bis 5, **dadurch gekennzeichnet, dass** das Übergangsmetall des Übergangsmetall-Katalysators Rhodium, Ruthenium, Platin, Palladium und/oder deren Salze ist.

7. Verfahren nach Anspruch 6, **dadurch gekennzeichnet, dass** der Übergangsmetall-Katalysator Ru-Titandioxid, Ru-Siliziumdioxid, R u-Aluminiumdioxid, Ru-Kohlenstoff, Pd-Kohlenstoff, Pd-Aktivkohle, Pd-Titandioxid, Pd-Siliziumdioxid, Pd-Aluminiumdioxid, Rh-Titandioxid, Rh-Siliziumdioxid, Rh-Kohlenstoff, Ir-Kohlenstoff, Ir-Titandioxid, Pt-Kohlenstoff, Pt-Aktivkohle, Pt-Titandioxid, Pt-Aluminiumoxid und/oder Pt-Siliziumdioxid ist.

8. Verfahren nach einem oder mehreren der Ansprüche 1 bis 7, **dadurch gekennzeichnet, dass** sich das Übergangsmetall auf einem Träger befindet, der aus Siliziumdioxid, Aluminiumoxid, Titandioxid, Zeolithe und/oder Kohlenstoff besteht.

9. Verfahren nach einem oder mehreren der Ansprüche 1 bis 8, **dadurch gekennzeichnet, dass** die Verbindungen der Formel (I) für 1,2,3,4-Tetrahydro-7-hydroxynaphthyl-1-on, 1,2,3,4-Tetrahydro-7-hydroxynaphthyl-2-on , 1 , 2 , 3 , 4-Tetrahydro-6-hydroxynaphthyl-1-on, 1,2,3,4-Tetrahydro-6-hydroxynaphthyl-2-on 1 , 2 , 3 , 4-Tetrahydro-5-hydroxynaphthyl-1-o n , 1 , 2 , 3 , 4-Tetrahydro-5-hydroxynaphthyl-2-on, 1,2,3,4-Tetrahydro-8-hydroxynaphthyl-1-on und 1,2,3,4-Tetrahydro-8-hydroxynaphthyl-2-on stehen.

10. Verfahren nach einem oder mehreren der Ansprüche 1 bis 9, **dadurch gekennzeichnet, dass** der Wasserstoff in situ erzeugt wird.

11. Verfahren nach einem oder mehreren der Ansprüche 1 bis 10, **dadurch gekennzeichnet, dass** die Reaktionstemperatur zwischen 120 °C und 170 °C liegt.

12. Verfahren nach einem oder mehreren der Ansprüche 1 bis 11, **dadurch gekennzeichnet, dass** der Reaktionsdruck zwischen 20 und 80 bar liegt.

13. Verfahren nach einem oder mehreren der Ansprüche 1 bis 12, **dadurch gekennzeichnet, dass** die Menge des Übergangsmetalls, bezogen auf die Verbindungen der Formel (II), zwischen 0,5 und 5 Gew. % beträgt.

14. Verfahren nach einem oder mehreren Ansprüchen 1 bis 13, **dadurch gekennzeichnet, dass** die Reaktion in Gegenwart einer Puffersubstanz durchgeführt wird.

## Claims

1. Process for the preparation of compounds of the formula (I) 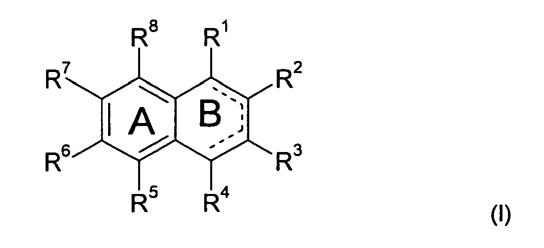 where ring B is completely or partially hydrogenated and
R¹, R², R³, R⁴, R⁵, R⁶, R⁷ or R⁸ may be identical or different and at least one of the radicals on ring A and on ring B is hydroxy and the other radicals are selected from the group C₁-C₁₅-alkyl, C₆-C₁₂-aryl, C₇-C₁₅-arylalkyl, C₁-C₁₅-alkylthio, C₁-C₁₅-alkoxy, hydroxy, hydrogen, C₁C₁₀-mono-or C₁-C₁₀-dialkylamino, C₁-C₁₅-haloalkyl, C₁-C₁₅-haloalkoxy, C₁-C₁₅-haloalkylthio, where halogen = F, Cl, Br, I, which may optionally be further substituted,
**characterized in that** compounds of the formula (II) 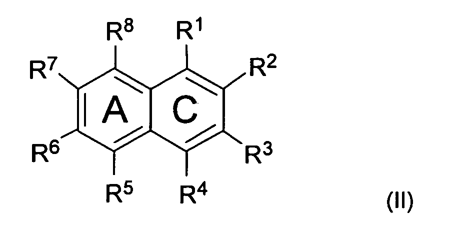 where R¹, R², R³, R⁴, R⁵, R⁶, R⁷ and R⁸ have the aforementioned meaning and at least one of the radicals on ring A and on ring C is hydroxy,
are reacted in the presence of at least one transition metal catalyst,
at least one organic, nonpolar, alicyclic or aromatic solvent
and hydrogen.

2. Process according to Claim 1, **characterized in that** R¹, R², R³, R⁴, R⁵, R⁶, R⁷ or R⁸ may be identical or different and are (C₁-C₆)-alkyl, (C₆-C₁₂)-aryl, (C₇-C₁₅)-arylalkyl, (C₁-C₆)-alkoxy, hydroxy, (C₁-C₆)-haloalkyl, (C₁-C₆)-haloalkoxy, (C₁-C₆)-haloalkylthio, (C₁-C₆)-alkylthio or hydrogen.

3. Process according to one of Claims 1 to 2, **characterized in that** ring A in compounds of the formula (I) is substituted by one or two hydroxyl groups in 6 and/or 7 position and ring B in compounds of the formula (I) is substituted by one or two hydroxyl groups in 2 and/or 3 position.

4. Process according to one of Claims 1 to 3, **characterized in that** ring B in compounds of the formula (I) is partially hydrogenated and one double bond is still present between the carbon atoms 1 and 2, 2 and 3, or 3 and 4 following the hydrogenation.

5. Process according to one or more of Claims 1 to 4, **characterized in that** the organic, nonpolar alicyclic or aromatic solvents are benzene, toluene, xylene, cyclohexane, or mixtures of such organic solvents.

6. Process according to one or more of Claims 1 to 5, **characterized in that** the transition metal in the transition metal catalyst is rhodium, ruthenium, platinum, palladium and/or salts thereof.

7. Process according to Claim 6, **characterized in that** the transition metal catalyst is Ru-titanium dioxide, Ru-silicon dioxide, Ru-aluminium dioxide, Ru-carbon, Pd-carbon, Pd-activated carbon, Pd-titanium dioxide, Pd-silicon dioxide, Pd-aluminium dioxide, Rh-titanium dioxide, Rh-silicon dioxide, Rh-carbon, Ir-carbon, Ir-titanium dioxide, Pt-carbon, Pt-activated carbon, Pt-titanium dioxide, Pt-aluminium oxide and/or Pt-silicon dioxide.

8. Process according to one or more of Claims 1 to 7, **characterized in that** the transition metal is located on a support which consists of silicon dioxide, aluminium oxide, titanium dioxide, zeolites and/or carbon.

9. Process according to one or more of Claims 1 to 8, **characterized in that** the compounds of the formula (I) are 1,2,3,4-tetrahydro-7-hydroxynaphthyl-1-one, 1,2,3,4-tetrahydro-7-hydroxynaphthyl-2-one, 1,2,3,4-tetrahydro-6-hydroxynaphthyl-1-one, 1,2,3,4-tetrahydro-6-hydroxynaphthyl-2-one, 1,2,3,4-tetrahydro-5-hydroxynaphthyl-1-one, 1,2,3,4-tetrahydro-5-hydroxynaphthyl-2-one, 1,2,3,4-tetrahydro-8-hydroxynaphthyl-1-one and 1,2,3,4-tetrahydro-8-hydroxynaphthyl-2-one.

10. Process according to one or more of Claims 1 to 9, **characterized in that** the hydrogen is produced in situ.

11. Process according to one or more of Claims 1 to 10, **characterized in that** the reaction temperature is between 120°C and 170°C.

12. Process according to one or more of Claims 1 to 11, **characterized in that** the reaction pressure is between 20 and 80 bar.

13. Process according to one or more of Claims 1 to 12, **characterized in that** the amount of transition metal, based on the compounds of the formula (II), is between 0.5 and 5% by weight.

14. Process according to one or more of Claims 1 to 13, **characterized in that** the reaction is carried out in the presence of a buffer substance.

## Revendications

1. Procédé pour la préparation de composés de
formule (I) 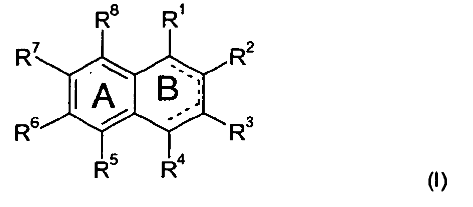 où le cycle B est totalement ou partiellement hydrogéné et
R¹ R², R³, R⁴, R⁵ , R⁶, R⁷ ou R⁸ peuvent être identiques ou différents et au moins un des radicaux sur le cycle A et sur le cycle B représente hydroxy et les autres radicaux sont choisis dans le groupe formé par C₁-C₁₅-alkyle, C₆-C₁₂-aryle, C₇-C₁₅-arylalkyle, C₁-C₁₅-alkylthio, C₁-C₁₅-alcoxy, hydroxy, hydrogène, C₁-C₁₀-monoalkylamino ou C₁-C₁₀-dialkylamino, C₁-C₁₅-halogénoalkyle, C₁-C₁₅-halogénoalcoxy, C₁-C₁₅-halogénoalkylthio, avec halogène = F, Cl, Br, I, qui peuvent le cas échéant être substitués davantage, **caractérisé**
**en ce qu'**on transforme des composés de formule (II) 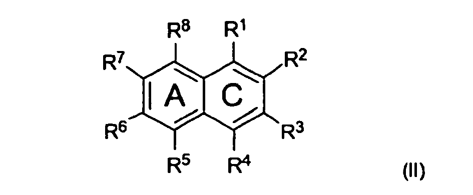 où
R¹, R², R³, R⁴, R⁵, R⁶, R⁷ et R⁸ présentent la
signification ci-dessus et au moins un des radicaux sur le cycle A et sur le cycle C représente hydroxy,
en présence d'au moins un catalyseur à base de métal de transition, d'au moins un solvant organique, non polaire, alicyclique ou aromatique et d'hydrogène.

2. Procédé selon la revendication 1, **caractérisé en ce que** R¹, R², R³, R⁴, R⁵, R⁶, R⁷ ou R⁸ peuvent être identiques ou différents et représentent (C₁-C₆)-alkyle, (C₆-C₁₂)-aryle, (C₇-C₁₅) -arylalkyle, (C₁-C₆) -alcoxy, hydroxy, (C₁-C₆)-halogénoalkyle, (C₁-C₆)-halogénoalcoxy, (C₁-C₆)-halogénoalkylthio, (C₁-C₆)-alkylthio ou hydrogène.

3. Procédé selon l'une quelconque des revendications 1 à 2, **caractérisé en ce que** le cycle A des composés de formule (I) est substitué par un ou deux groupes hydroxyle en position 6 et/ou 7 et le cycle B des composés de formule (I) est substitué par un ou deux groupes hydroxyle en position 2 et/ou 3.

4. Procédé selon l'une quelconque des revendications 1 à 3, **caractérisé en ce que** le cycle B des composés de formule (I) est partiellement hydrolysé et une double liaison existe encore entre les atomes de carbone 1 et 2, 2 et 3, ou 3 et 4 après l'hydrogénation.

5. Procédé selon l'une ou plusieurs des revendications 1 à 4, **caractérisé en ce qu'**il s'agit, pour les solvants organiques, non polaires, alicycliques ou aromatiques, de benzène, toluène, xylène, cyclohexane ou de mélanges de ces solvants organiques.

6. Procédé selon l'une ou plusieurs des revendications 1 à 5, **caractérisé en ce que** le métal de transition du catalyseur à base de métal de transition est le rhodium, le ruthénium, le platine, le palladium et/ou leurs sels.

7. Procédé selon la revendication 6, **caractérisé en ce que** le catalyseur à base de métal de transition est le dioxyde de Ru-titane, le dioxyde de Ru-silicium, le dioxyde de Ru-aluminium, le Ru-carbone, le Pd-carbone, le Pd-charbon actif, le dioxyde de Pd-titane, le dioxyde de Pd-silicium, le dioxyde de Pd-aluminium, le dioxyde de Rh-titane, le dioxyde de Rh-silicium, le Rh-carbone, l'Ir-carbone, le dioxyde d'Ir-titane, le Pt-carbone, le Pt-charbon actif, le dioxyde de Pt-titane, l'oxyde de Pt-aluminium et/ou le dioxyde de Pt-silicium.

8. Procédé selon l'une ou plusieurs des revendications 1 à 7, **caractérisé en ce que** le métal de transition se trouve sur un support qui est constitué par le dioxyde de silicium, l'oxyde d'aluminium, le dioxyde de titane, les zéolithes et/ou le carbone.

9. Procédé selon l'une ou plusieurs des revendications 1 à 8, **caractérisé en ce que** les composés de formule (I) sont la 1,2,3,4-tétrahydro-7-hydroxynaphtyl-1-one, la 1,2,3,4-tétrahydro-7-hydroxynaphtyl-2-one, la 1,2,3,4-tétrahydro-6-hydroxynaphtyl-1-one, la 1,2,3,4-tétrahydro-6-hydroxynaphtyl-2-one, la 1,2,3,4-tétrahydro-5-hydroxynaphtyl-1-one, la 1,2,3,4-tétrahydro-5-hydroxynaphtyl-2-one, la 1,2,3,4-tétrahydro-8-hydroxynaphtyl-1-one et la 1,2,3,4-tétrahydro-8-hydroxynaphtyl-2-one.

10. Procédé selon l'une ou plusieurs des revendications 1 à 9, **caractérisé en ce que** l'hydrogène est produit in situ.

11. Procédé selon l'une ou plusieurs des revendications 1 à 10, **caractérisé en ce que** la température de réaction se situe entre 120°C et 170°C.

12. Procédé selon l'une ou plusieurs des revendications 1 à 11, **caractérisé en ce que** la pression de réaction se situe entre 20 et 80 bars.

13. Procédé selon l'une ou plusieurs des revendications 1 à 12, **caractérisé en ce que** la quantité de métal de transition, par rapport aux composés de formule (II), est comprise entre 0,5 et 5% en poids.

14. Procédé selon l'une ou plusieurs des revendications 1 à 13, **caractérisé en ce que** la réaction est réalisée en présence d'une substance tampon.
